(19) Europäisches Patentamt
European Patent Office
Office européen des brevets

(11) Publication number: 0 104 432 A2

(12) **EUROPEAN PATENT APPLICATION**

(21) Application number: 83108306.8

(22) Date of filing: 23.08.83

(51) Int. Cl.³: A 01 N 31/02
A 01 N 33/20, A 01 N 35/02
A 01 N 35/04, A 01 N 37/30
A 01 N 37/34, A 01 N 37/36
A 01 N 37/42, A 01 N 37/48
A 01 N 39/00

(30) Priority: 25.08.82 JP 146276/82
06.01.83 JP 177/83

(43) Date of publication of application: 04.04.84 Bulletin 84/14

(84) Designated Contracting States: DE FR GB

(71) Applicant: TEIJIN LIMITED
11, 1-Chome, Minamihonmachi Higashi-ku
Osaka-shi Osaka(JP)

(72) Inventor: Hiramatsu, Toshiyuki
28-3, Ozu-cho 1-chome
Iwakuni-shi Yamaguchi-ken(JP)

(72) Inventor: Azuma, Shizuo
28-2, Ozu-cho, 1-chome
Iwakuni-shi Yamaguchi-ken(JP)

(72) Inventor: Yamaji, Teizo
5-10, Yamate-cho 4-chome
Iwakuni-shi Yamaguchi-ken(JP)

(74) Representative: Kraus, Walter, Dr. et al,
Patentanwälte Dres. Kraus & Weisert Irmgardstrasse 15
D-8000 München 71(DE)

(54) **Herbicide and plant growth regulator comprising alpha-beta-unsaturated compound.**

(57) A herbicide comprising as an active ingredient an α,β-unsaturated compound of the following formula (I)

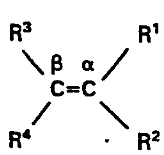

..... (I)

wherein

each of $R^1$ and $R^2$ represents CN, $NO_2$, a halogen, a $C_{1-25}$ alkoxy or alkylthio, a group of the formula

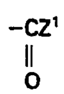

in which $Z^1$ represents $C_{1-25}$ alkyl or a phenyl, a group of the formula

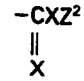

in which X's represent an oxygen or sulfur atom and $Z^2$ represents hydrogen, one equivalent of a cation, $C_{1-25}$ alkyl, a phenyl or a phenyl ($C_{1-25}$ alkyl), a group of the formula

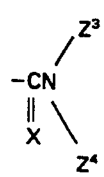

EP 0 104 432 A2

This invention relates to a herbicide and a plant growth regulator which comprises an α,β-unsaturated compound as an active ingredient.

The prior art has disclosed that α,β-unsaturated compounds can be the active ingredients of herbicides.

Assuming that α,β-unsaturated compounds are represented by the following formula

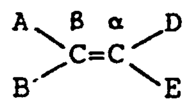

We will describe below those α,β-unsaturated compounds which have been known to be herbicidally active.

α,β-unsaturated compounds of the formula in which one of the groups (A, B) bonded to the carbon atom at the β-position is an amino or substituted amino group are described in Dutch Patent No. 6,813,063, Belgian Patents Nos. 817,744 and 835,646, U. S. Patents Nos. 3161643, 3452080, 3723498, 3726662, 3877924, 3981717, 4139700 and 4154599, Japanese Patent Publication No. 30821/1971, west German Patents Nos. 1924830 and 2330913, and British Patent No. 1,217,974.

α,β-Unsaturated compounds of the formula in which one (A) of the groups bonded to the carbon atom at the β-position is a hydrogen atom, the other (B) is a hydroxyl group or a halogen atom, and the group (D) bonded to the carbon atom at the α-position is a halogen atom are disclosed in French Patent No. 1,555,166 and U. S. Patent No, 3,492,111.

α,β-Unsaturated compounds of the formula in which one (A) of the groups bonded to the carbon atom at the β-position is a hydrogen atom, the other (B) is a phenyl or substituted phenyl group are disclosed in Japanese Laid-Open Patent Publication No. 53638/1978,

British Patent No. 2,060,634, U. S. Patent No. 3361790, Chim. Thér. 9, 347 (1974), Dutch Patent No. 6704128, and Bl, 1964, No. 10, pages 2612-2618.

Special α,β-unsaturated compounds of the formula in which one of the groups (A, B) bonded to the carbon atom at the β-position is a β-(1,2-epoxycyclohexan-1-yl)vinyl group are described in Japanese Patent Publication No. 8237/1972.

α,β-Unsaturated compounds of the formula in which the groups A and B bonded to the carbon atom at the β-position are bonded to each other to form a heterocyclic ring containing a nitrogen atom, or a sulfur atom or both are disclosed in U. S. Patents Nos. 3429895, 3634443, 3772331, 4020061 and 4052411 and Japanese Laid-Open Patent Publication No. 123,826/1975.

α,β-Unsaturated compounds of the formula in which the groups A and B bonded to the carbon atom at the β-position are both mercapto or lower alkylthio groups are disclosed in Japanese Patent Publication No. 21,120/1970.

α,β-Unsaturated compounds of the formula in which one of the groups A and B bonded to the carbon atom at the β-position is a halogen atom and the other is a halogen atom, a substituted mercapto group, an amino group, a substituted amino group or a heterocyclic ring are disclosed in U. S. Patent No. 3590068.

α,β-Unsaturated compounds of the formula in which either one of the groups (D and E) bonded to the carbon atom at the α-position is a substituted phenyl ether are disclosed in Japanese Laid-Open Patent Publication No. 139,622/1976.

α,β-Unsaturated compounds of the formula in which either one of the groups (D and E) bonded to the carbon atom at the α-position is a hydrogen atom are disclosed in U. S. Patents Nos. 3068230, 3136689, 3161495, 3213126, 3257422, 3370085, 3515538, 3597469, 3636082, 3772384 and 4052423, west German Patents Nos. 1793323

and 2729685, Japanese Patent Publications Nos. 15997/1965, 14318/1966, 31320/1970, and 22048/1966, Japanese Laid-Open Patent Publications Nos. 80234/1974, 135220/1975, 39623/1976, 64423/1977, and 154723/1979, Res. Discl. 154, 24 (1977), Farm. Ed. Sci., 28, 231 (1973), ibid. 28, 214 (1973), Sobre. Deriv. Cana. Azucar, 9, 38 (1975) and J. Agric. Food Chem. 25, 1413 (1977).

α,β-Unsaturated compounds of the formula in which either one of the groups (D and E) bonded to the carbon atom at the α-position is a dinitro-substituted phenyl ester residue are disclosed in U. S. Patent No. 3453318, British Patent No. 1,080,282 and Japanese Laid-Open Patent Publication No. 139341/1980.

$C_{2-3}$ acrylic acid esters of the formula in which either one of the groups (D and E) bonded to the carbon atom at the α-position is an ester residue including a phosphite or phosphate linkage are disclosed in U. S. Patents Nos. 3886236, 3980737, 3900536 and 3950457.

α,β-Unsaturated carboxylic acid esters of the formula in which either one of the groups (D and E) bonded to the carbon atom at the α-position is an amino-alkyl ester residue are disclosed in west German Patents Nos. 1,224,320 and 1,247,321.

U. S. Patent No. 3442638 discloses α,β-unsaturated carboxylic acid esters of the formula in which either one of the groups (D and E) bonded to the carbon atom at the α-position is an ester residue containing a mercury atom.

U. S. Patents Nos. 3169850 and 3278597, Japanese Patent Publication No. 16181/1967 and French Patent No. 2332706 disclose α,β-unsaturated carboxylic acid amides of the formula in which either one of the groups (D and E) bonded to the carbon atom at the α-position is a substituted or unsubstituted phenylamide residue.

The above-cited prior art references disclose α,β-unsaturated compounds as active ingredients of herbicides, and therefore, the present inventors believe

it preferable to acknowledge them herein as prior art against the herbicide of this invention.

The α,β-unsaturated compounds disclosed in the prior art references have their own characteristics in their chemical structure and herbicidal activity.

It is an object of this invention therefore to provide a herbicide comprising a specified α,β-unsaturated compound as a novel herbicidally active ingredient.

Another object of this invention is to provide a herbicide comprising as an active ingredient an α,β-unsaturated compound having a chemical structure in which the carbon atom at the α-position does not have a hydrogen atom but has two specified substituents.

Still another object of this invention is to provide a fast acting herbicide which when applied to the stalks or leaves of weeds, rapidly exhibits its herbicidal activity.

Still another object of this invention is to provide a herbicide having excellent absorbability which is very rapidly absorbed from the stalks or leaves of weeds, and with some types of its active ingredient, may begin to exhibit its herbicidal activity within 1 to 2 days after application.

Yet another object of this invention is to provide a herbicide having low animal and fish toxicity.

A further object of this invention is to provide a herbicide which exhibits excellent herbicidal activity against annual weeds.

A still further object of this invention is to provide a herbicide which exhibits excellent herbicidal activity against perennial weeds as well as annual weeds.

A yet further object of this invention is to provide a herbicide having excellent selectivity which exhibits strong herbicidal activity against either one of an annual broad-leaved weed and an annual narrow-leaved weed.

An additional object of this invention is to

provide a herbicide which can be produced very easily at low cost.

Other objects and advantages of this invention will become apparent from the following description.

According to this invention, these objects and advantages of the invention are achieved by a herbicide comprising as an active ingredient α,β-unsaturated compound represented by the following formula (I)

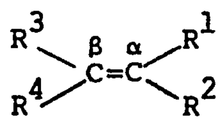
..... (I)

wherein

$R^1$ and $R^2$ are identical or different and each represents

CN,

$NO_2$,

a halogen atom,

a $C_{1-25}$ alkoxy or alkylthio group,

a group of the formula $-\underset{\overset{\parallel}{O}}{C}Z^1$ in which $Z^1$ represents a $C_{1-25}$ alkyl group or a phenyl group, which phenyl group may be substituted by 1 to 5 halogen atoms or 1 to 5 $C_{1-5}$ alkyl groups,

a group of the formula $-\underset{\overset{\parallel}{X}}{C}XZ^2$ in which X's, independently from each other, represent an oxygen or sulfur atom and $Z^2$ represents a halogen atom, one equivalent of a cation, a $C_{1-25}$ alkyl group, a phenyl group or a phenyl ($C_{1-25}$ alkyl) group, the phenyl moiety of which may be substituted by 1 to 5 halogen atoms or 1 to 5 $C_{1-5}$ alkyl groups, or

a group of the formula 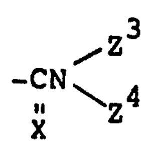 in which X is as defined above, and $Z^3$ and $Z^4$, independently from each other, represent a hydrogen atom, a

$C_{1-25}$ alkyl group, a phenyl($C_{1-25}$ alkyl) group or a triazolyl group, which triazolyl group may be substituted by a $C_{1-5}$ alkyl group, a phenyl group or a $C_{2-5}$ carboacyl group, or $Z^3$ and $Z^4$ may be bonded to each other to form a 5- or 6-membered ring together with the nitrogen atom to which they are bonded;

provided that $R^1$ and $R^2$ do not simultaneously represent the same member or the class consisting of $NO_2$, halogen atoms, $C_{1-25}$ alkoxy or alkylthio groups and groups $-\underset{X}{\overset{}{C}}Z^2$ in which $Z^2$ is a hydrogen atom;

$R^3$ represents

a group of the formula $Z^5X-$ in which $Z^5$ represents a hydrogen atom or a $C_{1-25}$ alkyl group and X is as defined above,

a group of the formula $Z^6O\underset{O}{\overset{\|}{C}}-$ in which $Z^6$ represents a hydrogen atom, one equivalent of a cation or a $C_{1-25}$ alkyl group,

a group of the formula $Z^7\underset{O}{\overset{\|}{C}}O-$ in which $Z^7$ represents a hydrogen atom or a $C_{1-25}$ alkyl group, or

a phenylthio, phenyl($C_{1-25}$ alkylthio) or phenyl-($C_{1-25}$ alkoxy) group, the phenyl moiety of which may be substituted by 1 to 5 halogen atoms or 1 to 5 $C_{1-5}$ alkyl groups;

$R^4$ represents

a hydrogen atom,

a $C_{1-25}$ alkyl group,

a 3- to 6-membered cycloalkyl group,

a phenyl($C_{1-25}$ alkyl) group, the phenyl moiety which may be substituted by 1 to 5 halogen atoms or 1 to 5 $C_{1-5}$ alkyl groups, or

the same groups as defined for $R^3$ above; and

$R^3$ and $R^4$ may be bonded to each other to form a 5-6-membered carbocyclic ring together with the carbon atom to which they are bonded;

provided that $R^3$ and $R^4$ do not simultaneously represent the same member of the class consisting of HO-, HS-, $C_{1-25}$ alkylthio groups, $HO\overset{O}{\overset{\|}{C}}-$, ($C_{1-25}$ alkyl)$\overset{O}{\overset{\|}{C}}O-$ and phenyl($C_{1-25}$ alkylthio) groups, and when $R^3$ is HO-, $R^4$ does not represent a hydrogen atom; and

further provided that $R^1$ or $R^2$ and $R^3$ or $R^4$ may be bonded to each other to form an acid anhydride group and that the $C_{1-5}$ alkyl and $C_{1-25}$ alkyl groups in the foregoing definitions may be interrupted by -O-, -S-, or $-NZ^8$ in which $Z^8$ represents a hydrogen atom or a $C_{1-10}$ alkyl group.

The α,β-unsaturated compound used as an active ingredient in this invention is represented by the above formula (I).

In formula (I), $R^1$ and $R^2$ may be identical or different. The halogen atom is, for example, fluorine, chlorine or bromine, and chlorine or bromine is preferred.

The $C_{1-25}$ alkoxy and $C_{1-25}$ alkylthio groups may be linear or branched. Examples of the $C_{1-25}$ alkoxy group include methoxy, ethoxy, n-propoxy, iso-propoxy, n-butoxy, iso-butoxy, sec-butoxy, tert-butoxy, n-pentoxy, n-hexoxy, n-octoxy, n-nonanoxy, n-decanoxy, n-undecanoxy, n-dodecanoxy, n-stearyloxy, n-eicosanoxy and n-pentacosanoxy. Of these, alkoxy groups having 1 to 10 carbon atoms are preferred, and those having 1 to 8 carbon atoms are especially preferred. Examples of the $C_{1-25}$ alkylthio groups include methylthio, ethylthio, n-propylthio, iso-propylthio, n-butylthio, sec-butylthio, iso-butylthio, tert-butylthio, n-pentylthio, n-hexylthio, n-octylthio, n-nonylthio, n-decylthio, n-undecylthio, n-dodecylthio, n-stearylthio, n-eicosylthio, and n-pentacosylthio. Of these, alkylthio groups having 1 to 18 carbon atoms

are preferred, and those having 1 to 10 carbon atoms are especially preferred.

The group $-\underset{\underset{O}{\|}}{C}Z^1$ (wherein $Z^1$ represents a $C_{1-25}$ alkyl group or a phenyl group, and the phenyl group may be substituted by 1 to 5 halogen atoms or 1 to 5 $C_{1-5}$ alkyl groups) represents $-\underset{\underset{O}{\|}}{C}\cdot C_{1-25}$ alkyl or $-\underset{\underset{O}{\|}}{C}\cdot$phenyl.

Examples of $-\underset{\underset{O}{\|}}{C}\cdot C_{1-25}$ alkyl includes acetyl, propionyl, butyryl, valeryl, caproyl, enathyl, caprilyl, pelargonyl, capryl, undecylyl, lauroyl, tridecanoyl, myristyl, pentadecanoyl, palmitoyl, margaryl, stearoyl, arachidyl, behenyl, and pentacosanoyl. Of these, $-\underset{\underset{O}{\|}}{C}\cdot C_{1-19}$ alkyl groups are preferred, and those having 1 to 11 carbon atoms in the alkyl moiety are especially preferred.

The halogen atom which can substitute the phenyl moiety of $-\underset{\underset{O}{\|}}{C}\cdot$phenyl is, for example, fluorine, chlorine or bromine, and fluorine and chlorine are preferred. The $C_{1-5}$ alkyl substituent is, for example, methyl, ethyl, propyl, butyl or pentyl.

Specific examples of the $-\underset{\underset{O}{\|}}{C}\cdot$phenyl include phenylcarbonyl, tolylcarbonyl, dimethylphenylcarbonyl, monoethylphenylcarbonyl, diethylphenylcarbonyl, isopropylphenylcarbonyl, tert-butylphenylcarbonyl, pentylphenylcarbonyl, monochlorophenylcarbonyl, monobromophenylcarbonyl, dichlorophenylcarbonyl, dibromophenylcarbonyl, trichlorophenylcarbonyl, 2-chloro-4-methylphenylcarbonyl, 2-bromo-4-methylphenylcarbonyl and 2-methyl-4-bromophenylcarbonyl.

Of these, phenylcarbonyl, mono- or di-halophenylcarbonyls, and monohalo-substituted mono-$C_{1-3}$ alkyl-substituted phenylcarbonyls are preferred.

The group $-\underset{\underset{O}{\parallel}}{C}XZ^2$ (in which $Z^2$ represents a hydrogen atom, one equivalent of a cation, a $C_{1-25}$ alkyl group, a phenyl group or a phenyl($C_{1-25}$ alkyl) group, and the phenyl moiety of the phenyl($C_{1-25}$ alkyl) group may be substituted by 1 to 5 halogen atoms or 1 to 5 $C_{1-5}$ alkyl groups, and X's, independently from each other, represent an oxygen or sulfur atom) include groups of the formulae $-\underset{\underset{O}{\parallel}}{C}-OZ^2$, $-\underset{\underset{O}{\parallel}}{C}-SZ^2$, $-\underset{\underset{S}{\parallel}}{C}-OZ^2$ and $-\underset{\underset{S}{\parallel}}{C}-SZ^2$.

Examples of one equivalent of a cation include an alkali metal such as sodium or potassium, 1/2 equivalent of an alkalin earth metal such as magnesium, calcium or barium, and ammonium ion of the following formula

$^{+}NH(R)_3$

wherein R's, independently from each other, represent a hydrogen atom, a linear or branched alkyl group having 1 to 18 carbon atoms, preferably an alkyl group having 1 to 14 carbon atoms, a 5- or 6-membered carbocyclic group such as cyclopentyl or cyclohexyl, or two R's may be bonded to each other to form a heterocyclic ring, such as a pyrrolidine, piperidine or morpholine ring, which may contain an additional nitrogen or oxygen atom.

The $C_{1-25}$ alkyl group may be linear or branched, and includes, for example, methyl, ethyl, n-propyl, isopropyl, n-butyl, iso-butyl, sec-butyl, tert-butyl, n-pentyl, n-hexyl, n-heptyl, sec-heptyl, n-octyl, n-nonyl, n-decyl, n-undecyl, n-dodecyl, n-tridecyl, n-tetradecyl, n-hexadecyl, n-stearyl, n-eicosyl, n-heneicosyl and pentacosyl. Of these, alkyl groups having 1 to 20 carbon atoms are preferred, and those having 1 to 15 carbon atoms are more preferred. Those having 1 to 10 carbon atoms are especially preferred.

Examples of the phenyl($C_{1-25}$ alkyl) groups are

benzyl, α-phenethyl, β-phenethyl, 1-phenylpropyl, 2-phenylpropyl, 1-phenylbutyl, 1-phenyldecyl, 1-phenyldodecyl, 1-phenylstearyl, 1-phenyleicosyl, and 1-phenylpentadecyl. Of these, phenyl($C_{1-12}$ alkyl) groups are preferred.

When $Z^2$ represents a hydrogen atom, one equivalent of a cation or a $C_{1-25}$ alkyl group, preferred examples of the group $-\underset{O}{\underset{\|}{C}}-OZ^2$ are a carboxyl group, $-COO\cdot C_{1-10}$ alkyl, $-CO\overset{-}{O}\overset{+}{H}_3\cdot C_{1-12}$ alkyl, and $-CO\overset{-}{O}\overset{+}{H}_4$.

Examples of the group $-\underset{O}{\underset{\|}{C}}-OZ^2$ in which $Z^2$ is a phenyl or phenyl($C_{1-25}$ alkyl) group include phenoxycarbonyl, benzyloxycarbonyl, monochlorobenzyloxycarbonyl, dichlorobenzyloxycarbonyl, monobromobenzyloxycarbonyl, dibromobenzyloxycarbonyl, α-phenethyl-oxycarbonyl, β-phenethyloxycarbonyl, 1-phenylpropyloxycarbonyl, 3-phenylpropyloxycarbonyl, ω-phenyldecyloxycarbonyl, ω-phenyldodecyloxycarbonyl, ω-phenylstearyloxycarbonyl and ω-phenylpentacosyloxycarbonyl. Of these, phenoxycarbonyl and phenyl($C_{1-18}$ alkyl)oxycarbonyl groups are preferred, and phenoxycarbonyl and phenyl($C_{1-12}$ alkyl)oxycarbonyl groups are especially preferred. The phenyl($C_{1-18}$ or $C_{1-12}$ alkyl)oxycarbonyl groups preferably have 1 to 5 halogen atoms as a substituent at the phenyl moiety.

Specific examples of the groups $-\underset{O}{\underset{\|}{C}}-SZ^2$, $-\underset{S}{\underset{\|}{C}}-OZ^2$ and $-\underset{S}{\underset{\|}{C}}-SZ^2$ will be easily understood from the examples of $Z^2$ and the examples of $-\underset{O}{\underset{\|}{C}}-OZ^2$ given above.

Examples of the group $-\underset{O}{\underset{\|}{C}}-SZ^2$ are mercaptocarbonyl and its salts, (methylthio)carbonyl, (ethylthio)-carbonyl, (phenylthio)carbonyl and (benzylthio)carbonyl. Examples of the group $-\underset{S}{\underset{\|}{C}}-OZ^2$ are hydroxy(thiocarbonyl)

and its salts, methoxy(thiocarbonyl), ethoxy(thiocarbonyl), phenoxy(thiocarbonyl) and benzyloxy(thiocarbonyl). Examples of $-\underset{\underset{S}{\|}}{C}-SZ^2$ are mercaptothiocarbonyl and its salts, methyldithiocarbonyl, ethyldithiocarbonyl, phenyldithiocarbonyl and benzyldithiocarbonyl.

The groups $-\underset{\underset{O}{\|}}{C}-OZ^2$ and $-\underset{\underset{O}{\|}}{C}-SZ^2$ are preferred as the group $-\underset{\underset{X}{\|}}{C}-XZ^2$.

The group $-\underset{\underset{X}{\|}}{C}-N\langle\begin{matrix}Z^3\\Z^4\end{matrix}$ (wherein $Z^3$ and $Z^4$, independently from each other, represent a hydrogen atom, a $C_{1-25}$ alkyl group, a phenyl($C_{1-25}$ alkyl) group or a triazoyl group, and the triazolyl group may be substituted by a $C_{1-5}$ alkyl group, a phenyl group or a $C_{2-5}$ carboacyl group, or $Z^3$ and $Z^4$ may be bonded to each other to form a 5- or 6-membered ring together with the nitrogen atom to which they are bonded and X is an oxygen or sulfur atom) includes groups of the formulae

$-\underset{\underset{O}{\|}}{C}-N\langle\begin{matrix}Z^3\\Z^4\end{matrix}$ and $-\underset{\underset{S}{\|}}{C}-N\langle\begin{matrix}Z^3\\Z^4\end{matrix}$.

Examples of the phenyl($C_{1-25}$ alkyl) and $C_{1-25}$ alkyl groups represented by $Z^3$ and $Z^4$ may be the same as given hereinabove with regard to $Z^2$. Examples of the $C_{1-5}$ alkyl group which can be a substituent on the triazolyl group are methyl, ethyl, propyl, butyl and pentyl. Examples of the $C_{2-5}$ carboacyl substituent are acetyl, propionyl, butyryl and valeryl.

Specific examples of the group $-\underset{\underset{O}{\|}}{C}-N\langle\begin{matrix}Z^3\\Z^4\end{matrix}$ include carbamoyl, monomethylcarbamoyl, dimethylcarbamoyl, monoethylcarbamoyl, diethylcarbamoyl, monopropylcarbamoyl, monoisobutylcarbamoyl, mono-sec-butylcarbamoyl, monopentylcarbamoyl, monodecylcarbamoyl, monolaurylcarbamoyl,

monostearylcarbamoyl, monopentacosylcarbamoyl, benzylcarbamoyl, dibenzylcarbamoyl, α-phenethylcarbamoyl, β-phenethylcarbamoyl, ω-phenyldecylcarbamoyl, ω-phenylstearylcarbamoyl, ω-phenylpentacosylcarbamoyl, 1,2,4-triazol-3-ylcarbamoyl, 5-phenyl-1,2,4-triazol-3-ylcarbamoyl, 5-methyl-1,2,4-triazol-3-ylcarbamoyl, 5-acetyl-1,2,4-triazol-3-ylcarbamoyl, pyrrolidylcarbonyl, piperidinocarbonyl, and morpholinocarbonyl.

Of these, carbamoyl, those groups of the above formula in which $Z^3$ and $Z^4$, independently from each other, are $C_{1-18}$ alkyl, particularly $C_{1-12}$ alkyl, phenyl($C_{1-18}$ alkyl), particularly phenyl($C_{1-12}$ alkyl), and triazolyl, and those groups of the above formula in which $Z^3$ and $Z^4$ are bonded to each other to form a pyrrolidine or piperidine ring together with the nitrogen atom to which they are bonded are preferred.

Examples of the group $-\underset{\underset{S}{\|}}{C}-N\begin{smallmatrix}Z^3\\ Z^4\end{smallmatrix}$ include thiocarbamoyl, monomethylthiocarbamoyl, dimethylthiocarbamoyl, monoethylthiocarbamoyl, diethylthiocarbamoyl, monopropylthiocarbamoyl, monoisobutylthiocarbamoyl, mono-sec-butylthiocarbamoyl, monopentylthiocarbamoyl, monodecylthiocarbamoyl, monolaurylthiocarbamoyl, monostearylthiocarbamoyl, monopentacosylthiocarbamoyl. benzylthiocarbamoyl, dibenzylthiocarbamoyl, α-phenethylthiocarbamoyl, β-phenethylthiocarbamoyl, ω-phenyldecylthiocarbamoyl, ω-phenylstearylthiocarbamoyl, ω-phenylpentacosylthiocarbamoyl, 1,2,4-triazol-3-ylthiocarbamoyl, 5-phenyl-1,2,4-triazol-3-ylthiocarbamoyl, 5-methyl-1,2,4-triazol-3-ylthiocarbamoyl, 5-acetyl-1,2,4-triazol-3-ylthiocarbamoyl, pyrrolidylthiocarbonyl, piperidinothiocarbonyl, and morpholinothiocarbonyl. Of these, thiocarbamoyl, those groups of the above formula in which $Z^3$ and $Z^4$, independently from each other, are $C_{1-18}$ alkyl, particularly $C_{1-12}$ alkyl, phenyl($C_{1-18}$ alkyl), particularly phenyl($C_{1-12}$ alkyl) and triazolyl, and those groups of the

above formula in which $Z^3$ and $Z^4$ are bonded to each other to form a pyrrolidine or piperidine ring together with the nitrogen atom to which they are bonded.

In the foregoing description of $R^1$ and $R^2$, the $C_{1-25}$ alkyl groups (including $C_{1-8}$ alkyl, $C_{1-12}$ alkyl, and $C_{1-5}$ alkyl) may be interrupted by -O-, -S- or $-\overset{|}{N}Z^8$ in which $Z^8$ is a hydrogen atom or a $C_{1-10}$ alkyl group.

Preferred $R^1$ and $R^2$ groups are CN, $NO_2$, halogen atoms, groups of the formula $-\underset{\underset{X}{\|}}{C}X-Z^2$ (in which $Z^2$ and X are as defined, provided that at least one of the two X's is an oxygen atom) and groups of the formula

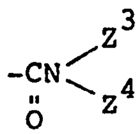 (in which $Z^3$ and $Z^4$ are as defined above).

It should be understood however that $R^1$ and $R^2$ do not simultaneously represent the same member of the class consisting of $NO_2$, halogen atoms and groups of formula $-\underset{\underset{X}{\|}}{C}XZ^2$ in which $Z^2$ is a hydrogen atom.

In general formula (I), $R^1$ or $R^2$ may be bonded to $R^4$ to form an acid anhydride group of the formula

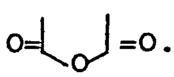. The acid anhydride group is also a preferred example in this invention.

With regard to $R^3$ of formula (I), $Z^5$-X (in which $Z^5$ represents a hydrogen atom or a $C_{1-25}$ alkyl group and X is an oxygen or sulfur atom) represents a hydroxyl group, a $C_{1-25}$ alkyloxy group, a mercapto group or a $C_{1-25}$ alkylthio group. Examples of the $C_{1-25}$ alkyl group may be the same as those given hereinabove.

$Z^6O\underset{\underset{O}{\|}}{C}-$ (in which $Z^6$ represents a hydrogen atom, one equivalent of a cation or a $C_{1-25}$ alkyl group) represents a carboxyl group (HOOC-), a carboxylate group (one equivalent of a cation·OOC-) and an ester residue ($C_{1-25}$ alkyl·OOC-).

Examples of one equivalent of a cation and $C_{1-25}$ alkyl are the same as those given hereinabove.

Specific examples of the carboxylate group and the ester residue are self-evident from the specific examples of one equivalent of a cation and $C_{1-25}$ alkyl given hereinabove, and are not given herein to avoid duplication.

A carboxyl group, $C_{1-10}$ alkyl·OOC-, $C_{1-12}$ alkyl-$\overset{+}{N}H_3OOC$, and $\overset{+}{N}H_4OOC$ are especially preferred as the group $Z^6O\underset{\overset{\|}{O}}{C}-$.

The group $Z^7\underset{\overset{\|}{O}}{C}O-$ (in which $Z^7$ represents a hydrogen atom or a $C_{1-25}$ alkyl group) represents $H\underset{\overset{\|}{O}}{C}O-$ or an acyloxy group of the formula $(C_{1-25}\ alkyl)\underset{\overset{\|}{O}}{C}O-$. Examples of the $C_{1-25}$ alkyl group may be the same as those given hereinabove. Of these, $(C_{1-10}\ alkyl)\underset{\overset{\|}{O}}{C}O-$ is is preferred, and $(C_{1-5}\ alkyl)\underset{\overset{\|}{O}}{C}O-$ is especially preferred.

The phenyl moiety of phenylthio, phenyl($C_{1-25}$ alkylthio) and phenyl($C_{1-25}$ alkoxy) may be substituted by 1 to 5 halogen atoms or 1 to 5 $C_{1-5}$ alkyl groups.

Examples of the phenyl($C_{1-25}$ alkylthio) include benzylthio, α-phenethylthio, β-phenethylthio, 1-phenyldodecylthio, 2-phenylpropylthio, 1-phenylbutylthio, 1-phenylpentylthio, 1-phenyldecylthio, 1-phenyldodecylthio, 1-phenylstearylthio, 1-phenyleicosylthio and 1-phenylpentacosylthio. Of these, phenyl($C_{1-5}$ alkylthio) groups are preferred.

The phenyl moiety of the above phenylthio, phenyl($C_{1-25}$ alkylthio) and phenyl($C_{1-25}$ alkoxy) groups may be substituted by 1 to 5 halogen atoms such as fluorine, chlorine or bromine or 1 to 5 $C_{1-5}$ alkyl groups such as methyl, ethyl, propyl, butyl or pentyl.

$R^4$ in formula (I) represents a hydrogen atom, a $C_{1-25}$ alkyl group, a 3- to 6-membered cycloalkyl group, a phenyl($C_{1-25}$ alkyl) group, or the same group as defined for $R^3$. Except the 3- to 6-membered cycloalkyl group, examples of the above groups for $R^4$ may be the same as those given hereinabove.

Examples of the 3- to 6-membered cycloalkyl group are cyclopropyl, cyclobutyl, cyclopentyl, and cyclohexyl. Cyclopropyl and cyclohexyl are preferred.

$R^3$ and $R^4$ may be bonded to each other to form a 5- or 6-membered carboxylic ring together with the carbon atom to which they are bonded. Examples of such a carbocyclic ring are a cyclopentane ring, a cyclohexane ring, a 3,3-ethylenedioxy-5,5-dimethylcyclohexane ring, and a 3-oxo-5,5-dimethylcyclohexane ring.

With regard to the description of $R^3$ and $R^4$, the $C_{1-25}$ alkyl groups (including $C_{1-10}$ alkyl and $C_{1-5}$ alkyl) may be interrupted by -O-, -S- or $-\overset{|}{N}Z^8$ (in which $Z^8$ represents a hydrogen atom or a $C_{1-10}$ alkyl group). Examples of the $C_{1-25}$ alkyl interrupted by such a hetero atom will be understood from the following Examples.

With regard to $R^3$ and $R^4$, $R^3$ is preferably the same as above, and $R^4$ is preferably a hydrogen atom, $C_{1-25}$ alkyl, $Z^{51}X$- (in which $Z^{51}$ is $C_{1-25}$ alkyl, and X is as defined above), or phenyl($C_{1-10}$ alkylthio). The $C_{1-25}$ alkyl may be interrupted by -O- or -S-, and $R^3$ and $R^4$ do not simultaneously represent the group of the class consisting of $C_{1-25}$ alkylthio groups and phenyl($C_{1-10}$ alkylthio) groups.

In the definitions of formula (I) or in the above preferred embodiments, some of the compounds of formula (I) are excluded from the scope of the invention by attaching the provisos.

When in formula (I) $R^3$ and $R^4$ are simultaneously HO or HOOC, or $R^1$ and $R^2$ simultaneously represent the group $-\underset{X}{\underset{\|}{C}}-XZ^2$ in which $Z^2$ is a hydrogen, the compounds of

of formula (I) are unstable and at least cannot be isolated.

When in formula (I), $R^3$ and $R^4$ simultaneously represent HS, $C_{1-25}$ alkylthio, phenyl($C_{1-25}$ alkyl)thio, or alkyl($C_{1-25}$)$\underset{\overset{\|}{O}}{C}$O-, or $R^3$ is HO- and $R^4$ is a hydrogen atom, or $R^1$ and $R^2$ simultaneously represent $NO_2$, halogen or $C_{1-25}$alkoxy or alkylthio, the compounds do not meet the objects of this invention because they have weak or insufficient herbicidal activity or their herbicidal activity is exhibited slowly.

Among the compounds of formula (I), preferred α,β-unsaturated compounds as herbicidally active ingredients are those of the following formula (I)-1

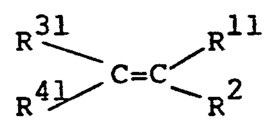

..... (I)-1

wherein

$R^{11}$ represents CN, a halogen atom, a group of the formula $-\underset{\overset{\|}{O}}{C}Z^1$ in which $Z^1$ is as defined, or a group of the formula $-CO\underset{\overset{\|}{O}}{Z^2}$ in which $Z^2$ is as defined above;

$R^2$ is as defined above;

provided that $R^{11}$ and $R^2$ do not simultaneously represent the same member of the class consisting of halogen atom and -COOH;

$R^{31}$ represents a group of the formula $Z^{51}X$- in which $Z^{51}$ and X are as defined above or a phenyl($C_{1-10}$ alkylthio) group;

$R^{41}$ represents a hydrogen atom, or a $C_{1-25}$ alkyl group which may be interrupted by -O- or -S-; and

$R^{31}$ and $R^{41}$ may be bonded to each other to form a 6-membered carbocyclic ring together with the carbon atom to which they are bonded.

Especially preferred α,β-unsaturated compounds of formula (I)-1 are those in which $R^2$ represents CN, $NO_2$, a halogen atom, a group of the formula $-\underset{\underset{X}{\|}}{C}XZ^2$ (in which $Z^2$ and X are as defined above, provided that at least one of the two X's is an oxygen atom), or a group of the formula $-\underset{\underset{O}{\|}}{C}N\begin{smallmatrix} Z^3 \\ Z^4 \end{smallmatrix}$ (in which $Z^2$ and $Z^3$ are as defined above), or $R^2$ may be bonded to $R^3$ or $R^4$ to form an acid anhydride group, provided that $R^{11}$ and $R^2$ do not simultaneously represent the same member of the class consisting of halogen atoms and -COOH.

Specific examples of the α,β-unsaturated compounds of formula (I) (including formula (I)-1) are given below. In the following exemplification, specific compounds are given in the order of $R^2$ being CN, $NO_2$, halogen, $C_{1-25}$ alkoxy or alkylthio, $-\underset{\underset{O}{\|}}{C}-Z^1$, $-\underset{\underset{X}{\|}}{C}-XZ^2$ and $-\underset{\underset{X}{\|}}{C}-N\begin{smallmatrix} Z^3 \\ Z^4 \end{smallmatrix}$ for each example of $R^1$. $R^3$ and $R^4$ are shown in a proper sequence as above for each specific example of $R^2$.

A. When $R^1$ is CN

A-1. $R^2$ = CN

(104) α-cyano-β-methoxyacrylonitrile,
(106) α-cyano-β-ethoxyacrylonitrile,
(108) α-cyano-β-n-propoxyacrylonitrile,
(110) α-cyano-β-isopropoxyacrylonitrile,
(112) α-cyano-β-n-butoxyacrylonitrile,
(114) α-cyano-β-sec-butoxyacrylonitrile,
(116) α-cyano-β-isobutoxyacrylonitrile,
(118) α-cyano-β-tert.butoxyacrylonitrile,
(120) α-cyano-β-n-pentoxyacrylonitrile,
(122) α-cyano-β-n-hexoxyacrylonitrile,
(124) α-cyano-β-n-heptoxyacrylonitrile,
(126) α-cyano-β-n-octoxyacrylonitrile,
(128) α-cyano-β-n-decanoxyacrylonitrile,
(129) α-cyano-β-n-dodecanoxyacrylonitrile,
(130) α-cyano-β-n-pentadecanoxyacrylonitrile,
(132) α-cyano-β-n-eicosanoxyacrylonitrile,
(134) α-cyano-β-mercaptoacrylonitrile,
(136) α-cyano-β-(methylthio)acrylonitrile,
(138) α-cyano-β-(ethylthio)accrylonitrile,
(140) α-cyano-β-(n-propylthio)acrylonitrile,
(142) α-cyano-β-(isopropylthio)acrylonitrile,
(144) α-cyano-β-(n-butylthio)acrylonitrile,
(146) α-cyano-β-(sec-butylthio)acrylonitrile,
(148) α-cyano-β-(isobutylthio)acrylonitrile,
(150) α-cyano-β-(tert.butylthio)acrylonitrile,
(152) α-cyano-β-(n-pentylthio)acrylonitrile,
(154) α-cyano-β-(n-decylthio)acrylonitrile,
(156) α-cyano-β-(n-stearylthio)cyanoacrylonitrile,
(164) α-cyano-β-hydroxycarbonylacrylonitrile,
(166) α-cyano-β-ethoxycarbonylacrylonitrile,
(168) α-cyano-β-n-butoxycarbonylacrylonitrile,
(170) α-cyano-β-tert.butoxycarbonylacrylonitrile,
(172) α-cyano-β-hydroxycarbonylacrylonitrile

monosodium salt,

(174) α-cyano-β-formyloxyacrylonitrile,

(176) α-cyano-β-acetyloxyacrylonitrile,

(178) α-cyano-β-propionyloxyacrylonitrile,

(180) α-cyano-β-phenylthioacrylonitrile,

(182) α-cyano-β-methyl-β-ethoxyacrylonitrile,

(184) α-cyano-β-ethyl-β-ethoxyacrylonitrile,

(186) α-cyano-β-methyl-β-isopropoxyacrylonitrile,

(188) α-cyano-β-n-decyl-β-ethoxyacrylonitrile,

(190) α-cyano-β-n-dodecyl-β-ethoxyacrylonitrile,

(192) α-cyano-β-cyclohexyl-β-ethoxyacrylonitrile.

A-2. $R^2 = NO_2$

(196) α-nitro-β-ethoxyacrylonitrile,

(198) α-nitro-β-n-decanoxyacrylonitrile,

(200) α-nitro-β-mercaptoacrylonitrile,

(202) α-nitro-β-methylthioacrylonitrile,

(204) α-nitro-β-(ethylthylthio)acrylonitrile,

(206) α-nitro-β-(n-butylthio)acrylonitrile,

(212) α-nitro-β-hydroxycarbonylacrylonitrile,

(214) α-nitro-β-ethoxycarbonylacrylonitrile,

(216) α-nitro-β-acetyloxyacrylonitrile,

(218) α-nitro-β-(phenylthio)acrylonitrile,

(220) α-nitro-β-methyl-β-ethoxyacrylonitrile,

(222) α-nitro-β-ethyl-β-ethoxyacrylonitrile,

(224) α-nitro-β-n-decyl-β-ethoxyacrylonitrile,

(226) α-nitro-β-cyclohexyl-β-ethylthioacrylonitrile.

A-3. $R^2$ = halogen

(228) α-chloro-β-methoxyacryloniitrile,

(232) α-bromo-β-methoxyacrylonitrile,

(234) α-bromo-β-ethoxyacrylonitrile,

(236) α-bromo-β-mercaptoacrylonitrile,

(238) α-bromo-β-(methylthio)acrylonitrile,

(240) α-bromo-β-cyclohexylacrylonitrile,

(242) α-bromo-β-(phenylthio)acrylonitrile,
(244) α-bromo-β-methyl-β-ethoxyacrylonitrile,
(246) α-bromo-β-ethyl-β-ethoxyacrylonitrile,
(248) α-bromo-β-decyl-β-ethoxyacrylonitrile,
(250) α-bromo-β-ethyl-β-(ethylthio)acryonitrile,
(252) α-bromo-β-ethyl-β-(phenylthio)acrylonitrile,

A-4. $R^2$ = alkoxy or alkylthio

(262) α,β-diethoxyacrylonitrile,
(264) α-ethoxy-β-n-butoxyacrylonitrile,
(266) α-ethoxy-β-(ethylthio)acrylonitrile,
(268) α-ethoxy-β-(phenylthio)acrylonitrile,
(270) α-ethoxy-β-ethoxycarbonylacrylonitrile,
(272) α-ethoxy-β-acetyloxyacrylonitrile,
(274) α-ethoxy-β-cyclohexylacrylonitrile,
(276) α-ethoxy-β-hydroxycarbonylacrylonitrile,
(278) α-ethoxy-β-ethoxycarbonylacrylonitrile,
(280) α-ethoxy-β-acetyloxyacrylonitrile,
(282) α,β-diethoxy-β-methylacrylonitrile.

A-5. $R^2 = -\underset{\underset{O}{\|}}{C}Z^1$

(294) α-benzoyl-β-ethoxyacrylonitrile,
(296) α-benzoyl-β-mercaptoacrylonitrile,
(298) α-benzoyl-β-(ethylthio)acrylonitrile,
(300) α-benzoyl-β-(phenylthio)acrylonitrile,
(302) α-acetyl-β-methyl-β-ethoxyacrylonitrile,
(304) α-acetyl-β-methyl-β-(ethylthio)acrylonitrile,
(306) α-benzoyl-β-ethyl-β-(phenylthio)acrylonitrile.

A-6. $R^2 = -\underset{\underset{O}{\|}}{C}OZ^2$

(310) α-cyano-β-cyclopropyl acrylic acid,
(312) α-cyano-β-cyclohexyl acrylic acid,
(314) α-cyano-β-methoxy acrylic acid,

(316) α-cyano-β-methoxy acrylic acid ethyl ester,
(318) α-cyano-β-ethoxy acrylic acid ethyl ester,
(320) α-cyano-β-ethoxy acrylic acid isobutyl ester,
(322) α-cyano-β-n-propoxy acrylic acid ethyl ester,
(324) α-cyano-β-n-butoxy acrylic acid ethyl ester,
(326) α-cyano-β-n-pentoxy acrylic acid ethyl ester,
(328) α-cyano-β-mercapto acrylic acid ethyl ester,
(330) α-cyano-β-(ethylthio)acrylic acid ethyl ester,
(332) α-cyano-β-(n-propylthio)acrylic acid ethyl ester,
(334) α-cyano-β-(n-butylthio)acrylic acid ethyl ester,
(336) α-cyano-β-(n-pentylthio)acrylic acid ethyl ester,
(338) α-cyano-β-(n-octylthio)acrylic acid ethyl ester,
(334) α-cyano-β-acetyloxy acrylic acid ethyl ester,
(345) α-cyano-β-ethoxy acrylic acid phenyl ester,
(346) α-cyano-β-(ethylthio)acrylic acid phenyl ester,
(347) α-cyano-β-(phenylthio)acrylic acid ethyl ester,
(348) α-cyano-β-(benzylthio)acrylic acid ethyl ester,
(349) α-cyano-β-methyl-β-ethoxy acrylic acid isobutyl ester,
(350) α-cyano-β-methyl-β-ethoxy acrylic acid ethyl ester,
(352) α-cyano-β-ethyl-β-ethoxy acrylic acid ethyl ester,
(353) α-cyano-β-n-propyl-β-ethoxy acrylic acid ethyl ester,
(354) α-cyano-β-n-dodecyl-β-ethoxy acrylic acid ethyl ester,
(356) α-cyano-β-methyl-β-(ethylthio)acrylic acid ethyl ester,

(357) α-cyano-β-methoxy-β-(thylthio)acrylic acid ethyl ester,

(358) Ethyl (3-ethylenedioxy-5,5-dimethylcyclohexylidene)-α-cyanoacetate,

(359) α-cyano-β-cyclohexyl-β-ethoxy acrylic acid ethyl ester.

A-7. $R^2 = \underset{X}{\overset{\|}{C}}XZ^2$ (at least one of X's is S)

(360) α-cyano-β-ethylthio thioacrylic acid ethyl ester,

(362) α-cyano-β-ethoxy thioacrylic acid ethyl ester,

(364) α-cyano-β-ethylthio thioacrylic acid phenyl ester,

(365) α-cyano-β-ethoxy thioacrylic acid phenyl ester,

(366) α-cyano-β-n-butylthio thioacrylic acid phenyl ester,

(368) α-cyano-β-ethoxy thioacrylic acid benzyl ester,

(370) α-cyano-β-n-butoxy thioacrylic acid benzyl ester,

(372) α-cyano-β-ethylthio thioacrylic acid benzyl ester,

(374) α-cyano-β-n-butylthio thioacrylic acid benzyl ester,

(375) α-cyano-β-(ethoxythiocarbonyl)-β-ethylthio ethylene,

(376) α-cyano-β-ethoxy dithioacrylic acid ethyl ester,

(378) α-cyano-β-ethylthio dithioacrylic acid ethyl ester,

(380) α-cyano-β-methyl-β-ethoxy thioacrylic acid phenyl ester,

(382) α-cyano-β-ethyl-β-ethoxy thioacrylic acid phenyl ester

(384) α-cyano-β-ethyl-β-ethoxy dithioacrylic acid ethyl ester.

A-8. $R^2 = -\underset{\underset{X}{\|}}{C}N{<}^{Z^3}_{Z^4}$

(390) α-cyano-β-n-butoxy acrylic acid sec.butylamide,

(392) α-cyano-β-ethylthio acrylic acid sec.butylamide,

(394) α-cyano-β-n-butylthio acrylic acid sec.butylamide,

(396) α-cyano-β-ethoxy acrylic acid piperazide,

(400) α-cyano-β-n-butoxy acrylic acid piperazide,

(402) α-cyano-β-ethylthio acrylic acid piperazide,

(404) α-cyano-β-n-butylthio acrylic acid piperazide,

(406) α-cyano-β-ethoxy acrylic acid 1,2,4-triazolylamide,

(408) α-cyano-β-ethoxy acrylamide,

(410) α-cyano-β-ethylthio acrylamide,

(412) α-cyano-β-ethoxythioacrylamide.

B. <u>When $R^1$ is $NO_2$</u>

B-1. $R^2$ = halogen

(420) α-nitro-α-chloro-β-ethoxy ethylene,

(422) α-nitro-α-bromo-β-ethoxy ethylene,

(424) α-nitro-α-bromo-β-ethylthio ethylene.

B-2. $R^2$ = alkoxy or alkylthio

(426) α-nitro-α-methoxy-β-ethylthio ethylene,

(428) α-nitro-α,β-diethoxy ethylene,

(430) α-nitro-α-methoxy-β-benzylthio ethylene.

B-3. $R^2 = -\underset{\underset{X}{\|}}{C}Z^1$

(432) α-nitro-β-acetyl-β-ethoxy ethylene,

(434) α-nitro-β-benzoyl-β-ethylthio ethylene.

B-4. $R^2 = -\underset{\underset{X}{\|}}{C}XZ^2$

(436) α-nitro-β-cyclohexyl-β-ethoxy acrylic acid,

(440) α-nitro-β-ethoxy acrylic acid ethyl ester,

(442) α-nitro-β-ethylthio acrylic acid ethyl ester,

(444) α-nitro-β-acetyloxy acrylic acid ethyl ester,

(446) α-nitro-β-ethoxy thioacrylic acid,

(448) α-nitro-β-ethylthio thioacrylic acid,

(450) α-nitro-β-ethoxy thioacrylic acid ethyl ester,

(452) α-nitro-β-ethylthio thioacrylic acid ethyl ester,

(454) α-nitro-β-methyl-β-ethoxy acrylic acid ethyl ester,

(456) α-nitro-β-ethyl-β-ethylthio acrylic acid ethyl ester.

B-5. $R^2 = -\underset{\underset{X}{\|}}{C}N\genfrac{}{}{0pt}{}{\diagup Z^3}{\diagdown Z^4}$

(460) α-nitro-β-ethoxy acrylamide,

(462) α-nitro-β-ethylthio acrylamide.

<u>C. When $R^1$ is a halogen atom</u>

C-1. $R^2$ = alkoxy, alkylthio or $-\underset{\underset{O}{\|}}{C}Z^1$

(470) α-bromo-α-methoxy-β-ethoxy ethylene,

(472) α-bromo-α-ethylthio-β-ethoxy ethylene,

(474) α-bromo-α-acetyl-β-ethoxy ethylene.

C-2. $R^2 = -\underset{\underset{X}{\|}}{C}XZ^2$

(476) α-bromo-β-phenylthio acrylic acid,

(478) α-bromo-β-cyclohexyl-β-ethylthio acrylic acid

ethyl ester,

(480) α-bromo-β-acetyloxy acrylic acid ethyl ester,

(482) α-bromo-β-ethoxy acrylic acid ethyl ester,

(484) α-bromo-β-ethylthio acrylic acid ethyl ester,

(486) α-bromo-β-phenylthio acrylic acid ethyl ester.

C-3. $R^2$ = 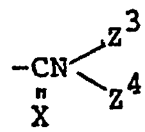

(438) α-bromo-β-ethoxy acrylamide,

(490) α-chloro-β-methylthio acrylamide,

(492) α-chloro-β-ethoxy acrylic acid sec.butylamide.

D. When $R^1$ is alkoxy or alkylthio and $R^2$ is $-\underset{O}{\overset{\|}{C}}Z^1$, $-\underset{X}{\overset{\|}{C}}XZ^2$ or $-\underset{X}{\overset{\|}{C}}N\begin{matrix}Z^3\\Z^4\end{matrix}$

(494) α-methoxy-β-ethoxy acrylamide,

(496) α-ethoxy-α-acetyl-β-ethoxy ethylene,

(498) α-ethylthio-β-ethoxy acrylic acid ethyl ester,

(500) α,β-diethoxy acrylic acid ethyl ester,

(502) α-methoxy-β-ethoxy thioacrylic acid ethyl ester.

E. When $R^1$ is $-\underset{O}{\overset{\|}{C}}Z^1$, and $R^2$ is $-\underset{O}{\overset{\|}{C}}Z^1$, $-\underset{X}{\overset{\|}{C}}XZ^2$ or

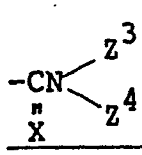

(504) α-acetyl-β-ethoxy acrylic acid ethyl ester,

(506) α-acetyl-β-butoxy acrylic acid ethyl ester,

(508) α-benzyl-β-ethoxy acrylamide,

(510) α-acetyl-β-methoxy thioacrylic acid ethyl ester.

F. When $R^1$ is $-CXZ^2$, and $R^2$ is carbalkoxy or
X

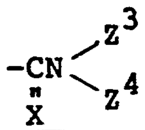

(512) α-ethoxycarbonyl-β-ethoxy acrylic acid ethyl ester,

(514) α-ethoxycarbonyl-β-ethylthio acrylic acid ethyl ester,

(515) α-ethoxycarbonyl-β-ethoxy thioacrylic acid ethyl ester,

(516) α-ethoxycarbonyl-β-ethoxy acrylic acid ethyl ester,

(517) α-ethoxycarbonyl-β-ethoxy acrylamide,

(518) α,β-diethoxycarbonyl-β-hydroxy acrylamide.

G. When both $R^1$ and $R^2$ are 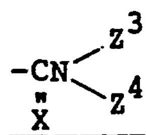

(520) 2-ethoxymethylene malonic acid di-sec.butyl amide,

(522) 2-ethoxymethylene malondiamide.

H. Others

(524) α-chloromaleic acid anhydride,

(526) α-cyano-β-methyl maleic acid anhydride.

The α,β-unsaturated compound of formula (I) can be produced by any one of the following methods according to specified groups of $R^1$, $R^2$, $R^3$ and $R^4$. These methods are known *per se*.

A first method is a condensation reaction shown by the following scheme.

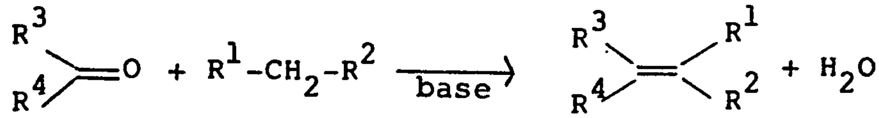


This method can give α,β-unsaturated compounds of formula (I) in which $R^3$ and $R^4$ represent a hydrogen atom, a $C_{1-25}$ alkyl group, a 3- to 6-membered cycloalkyl group or a phenyl($C_{1-25}$ alkyl) group, or $R^3$ and $R^4$ are bonded to each other to form a 5- or 6-membered carbocyclic ring, and $R^1$ and $R^2$ represent CN, $NO_2$, 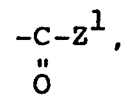,

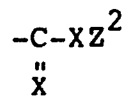 or 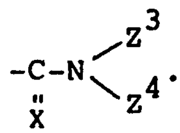.

A second method is a condensation reaction shown by the following scheme.

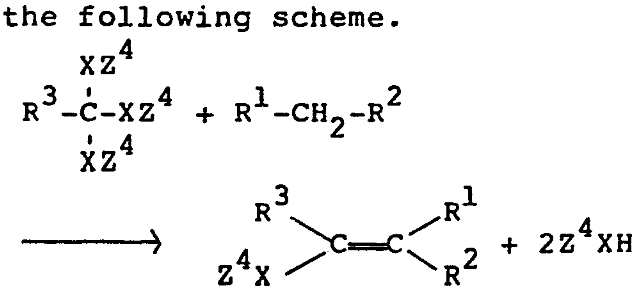

This method can give α,β-unsaturated compounds of general formula (I) in which $R^3$ represents a hydrogen atom, a $C_{1-25}$ alkyl group, a 3- to 6-membered cycloalkyl group or a phenyl($C_{1-25}$ alkyl group, $R^4$ is $Z^5X$, and

$R^1$ and $R^2$ represent CN, $NO_2$, 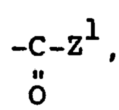, 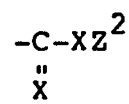 or 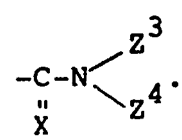.

A third method is a reaction shown by the following scheme.

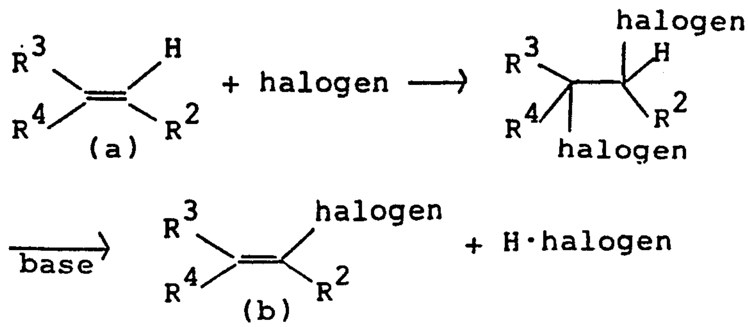

The third method can give α,β-unsaturated compounds (b) corresponding to general formula (I) in which $R^1$ is a halogen atom.

When a compound of the following formula

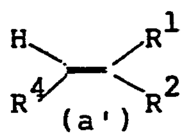

(a')

is used instead of the compound of formula (a) in the third method, α,β-unsaturated compounds of the following formula corresponding to formula (I) in which $R^3$ is a halogen atom can be obtained.

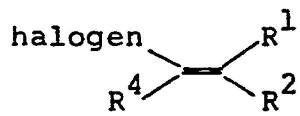

(b')

Alcoholysis (or thioalcoholysis) of the compounds of formula (b) or the compounds of formula (b') can give α,β-unsaturated compounds of formula (I) in which $R^1$ is an alkoxy or alkylthio group having 1 to 25 carbon atoms, or α,β-unsaturated compounds of formula (I) in which $R^3$ represents $Z^5X-$, a phenyl($C_{1-25}$ alkylthio) group or a phenylthio group.

A fourth method is the hydrolysis of the product obtained by the second method, which yields an α,β-unsaturated compound of formula (I) in which $R^4$ is HO or HS.

By reacting the resulting compound in which $R^4$ is HO with an acyl halide such as $Z^7\underset{\underset{O}{\|}}{C}Cl$, α,β-unsaturated compounds in which $R^4$ is $Z^7\underset{\underset{O}{\|}}{C}O-$ can be produced.

A fifth method is a method for producing compounds of formula (I) in which $R^3$ is $Z^6O\underset{\underset{O}{\|}}{C}-$ or $R^4$ and $R^2$ are bonded to each other to form an acid anhydride group.

This method is schematically shown as follows:

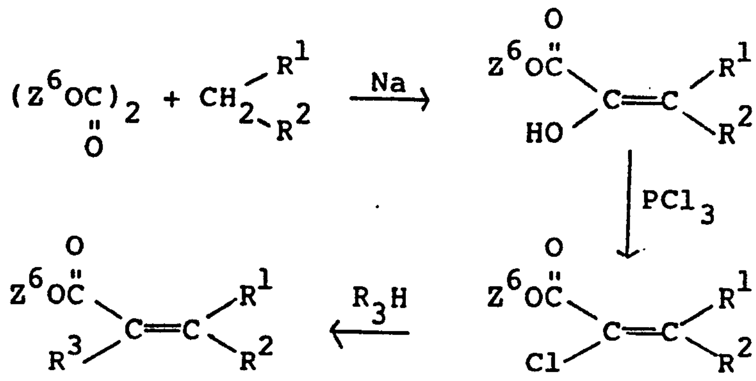


(provided that $R^3$ is not $Z^6O\underset{O}{\underset{\|}{C}}$)

By treating in a customary manner, the final compound in which $R^2$ is $\underset{O}{\underset{\|}{C}}OZ^6$ obtained by the above reaction, a compound of formula (I) in which $R^4$ and $R^2$ are bonded to each other to form an acid anhydride group is produced.

The α,β-unsaturated compounds represented by formula (I) show excellent herbicidal activity and are used for combating weeds.

The weeds herein denote useless or hazardous plants which grow in environments created by man such as paddies and upland farms after they have come into these environments from the surrounding nature.

Examples of the weeds include plants of the family Gramineae such as those of the genera Digitaria, Sorghum and Echinochloa; plants of the family Plantaginaceae such as genus Plantago; plants of the family Cyperaceae such as those of the genus Cyperus; plants of the family Compositae such as those of the genus Erigeron; plants of the family Cruciferae such as Barbarae vulgaris and those of the genus Raphanus; plants of the family Solanaceae such as those of the genus Physalis; plants of the family Commelinaceae such as those of the genus Commelina; and plants of the family Leguminosae such as those of the genus Medicago.

The active compounds in accordance with this invention greatly affect the growth of plants, and therefore can control the growth of plants when applied in amounts smaller than those normally used in controlling weeds although the amounts may vary depending upon the kinds of the active compounds.

The active compounds of this invention can be applied to weeds or their habitat at a rate of 10 g to 200 kg, preferably 50 to 100 kg, per hectare.

The active compounds of this invention may be used in usual types of formulations such as solutions, emulsifiable concentrates, suspensions, dusts, pastes, or granules. These formulations may be prepared by using one or more of various adjuvants, for example, solid carriers such as talc, bentonite, clay, kaolin, diatomaceous earth, white carbon, vermiculite, slaked lime, ammonium sulfate and urea; liquid carriers such as water, alcohols, dioxane, acetone, xylene, cyclohexane, methylnaphthalene and dimethylformamide; surface-active agents, emulsifiers, dispersants and stickers such as alkyl sulfates, alkylsulfonic acid salts, ligninsulfonic acid salts, polyoxyethylene glycol ethers, polyoxyethylene alkylaryl ethers, polyoxyethylene sorbitan monoalkylates, and dinaphthylmethanedisulfonic acid salts; and various other adjuvants such as carboxymethyl cellulose and gum arabic.

These formulations may be prepared, for example, by mixing the active compound with a carrier and/or an emulsifier exemplified above.

The active compound of this invention can be present usually in an amount of 0.01 to 99% by weight, preferably 0.1 to 95% by weight, in such a formulation.

The active compound of this invention may be applied singly or as a mixture with another active compound or in each of the formulations exemplified above to plants by usual methods such as spraying, atomizing, scattering and dusting.

The active compounds of this invention are characterized by being rapidly absorbed from the stalks or leaves of weeds and rapidly exhibit their herbicidal activity or plant growth modifying activity.

The active compounds of this invention include those which exhibit excellent activity against annual weeds, those which exhibit excellent activity against parennial weeds as well as annual weeds, and those which exhibit selective strong activity against one of an annual broad-leaved weed and an annual narrow-leaved weed.

The following Examples illustrate the present invention in more detail.

All parts in these examples are parts by weight. The herbicidal activity of each herbicide was evaluated on a scale of 1 to 5 wherein "1" means that weeds were as alive as they had been before the application of the herbicide; "5" means that weeds entirely withered as a result of applying the herbicide; and 2, 3 and 4 mean varying degrees of the weakened states of weeds between 1 and 5.

Preparation Example

Some active compounds in accordance with this invention were prepared, and their physical properties are shown in Table 1.

Table 1

| Compound No. | IR | | NMR | |
|---|---|---|---|---|
| | $\nu_{max}(cm^{-1})$ | Assignment | Solvent | δ (ppm) |
| 106 | 2240<br>1600 | Cyano<br>Vinylene | $CDCl_3$ | 7.30 (t, 1H)<br>2.58 (m, 2H)<br>1.32 (br, 10H)<br>0.90 (br, t, 3H) |
| 112 | 2240<br>1600 | Cyano<br>Vinylene | $CCl_4$ | 7.56 (s, 1H)<br>4.30 (t, 2H)<br>2.1-1.2 (m, 4H) |
| 138 | 2230<br>1520 | Cyano<br>Vinylene | $CCl_4$ | 1.00 (t, 3H)<br>8.06 (s, 1H)<br>3.10 (q, 2H)<br>1.13 (t, 3H) |
| 144 | 2220<br>1510 | Cyano<br>Vinylene | $CCl_4$ | 8.15 (s, 1H)<br>3.05 (t, 2H)<br>2.0-0.8 (m, 4H)<br>1.0 (t, 3H) |
| 182 | 2220<br>1570 | Cyano<br>Vinylene | $CCl_4$ | 4.15 (q, 2H)<br>2.40 (s, 3H)<br>1.50 (t, 3H) |
| 184 | 2230<br>1560 | Cyano<br>Vinylene | $CCl_4$ | 4.50 (q, 2H)<br>2.60 (q, 2H)<br>1.46 (t, 3H)<br>1.26 (t, 3H) |
| 190 | 2210<br>1560 | Cyano<br>Vinylene | $CCl_4$ | 4.45 (q, 2H)<br>2.55 (t, 2H)<br>0.7-1.7 (m, 26H) |
| 312 | 2220<br>1680 | Cyano<br>Carbonyl | $CDCl_3$ | 9.70 (s, 1H)<br>7.55 (d, 1H)<br>2.3-3.0 (m, 1H)<br>0.9-2.0 (m, 10H) |

- to be continued -

Table 1 (continued)

| Compound No. | IR |  | NMR |  |
|---|---|---|---|---|
|  | $\nu_{max}(cm^{-1})$ | Assignment | Solvent | δ (ppm) |
| 316 | - | - | $CCl_4$ | 8.05 (s, 1H)<br>4.22 (q, 2H)<br>3.40 (s, 3H)<br>1.17 (t, 3H) |
| 318 | - | - | $CCl_4$ | 7.90 (s, 1H)<br>4.40 (q, 2H)<br>4.26 (q, 2H)<br>1.50 (t, 3H)<br>1.26 (t, 3H) |
| 320 | - | - | $CDCl_3$ | 7.70 (s, 1H)<br>4.28 (q, 2H)<br>3.95 (d, 2H)<br>2.00 (m, 1H)<br>1.45 (t, 3H)<br>1.00 (d, 6H) |
| 322 | - | - | $CCl_4$ | 7.90 (s, 1H)<br>4.26 (t, 2H)<br>4.20 (q, 2H)<br>1.4-2.1 (m, 2H)<br>1.30 (t, 3H)<br>1.03 (t, 3H) |
| 324 | - | - | $CCl_4$ | 7.85 (s, 1H)<br>4.30 (t, 2H)<br>4.20 (q, 2H)<br>1.1-1.9 (m, 4H)<br>1.32 (t, 3H)<br>1.00 (t, 3H) |

- to be continued -

Table 1 (continued)

| Compound No. | IR | | NMR | |
|---|---|---|---|---|
| | $\nu_{max}(cm^{-1})$ | Assignment | Solvent | δ (ppm) |
| 326 | - | - | $CCl_4$ | 7.90 (s, 1H)<br>4.63 (t, 2H)<br>4.60 (q, 2H)<br>1.1-1.9 (m, 6H)<br>1.30 (t, 3H)<br>0.85 (t, 3H) |
| 344 | - | - | $CCl_4$ | 8.20 (s, 1H)<br>4.20 (q, 2H)<br>1.95 (s, 3H)<br>1.30 (t, 3H) |
| 350 | - | - | $CDCl_3$ | 4.28 (q, 2H)<br>3.92 (q, 2H)<br>2.62 (s, 3H)<br>1.52 (t, 3H)<br>1.28 (t, 3H) |
| 352 | - | - | $CDCl_3$ | 4.40 (q, 2H)<br>4.26 (q, 2H)<br>3.02 (q, 2H)<br>1.15-1.52 (m, 9H) |
| 354 | 2200<br>1725<br>1560 | Cyano<br>Carbonyl<br>Vinylene | $CCl_4$ | 3.9-4.4 (m, 4H)<br>2.20 (t, 2H)<br>0.7-1.7 (m, 29H) |
| 356 | - | - | $CDCl_3$ | 7.70 (s, 1H)<br>4.20 (q, 2H)<br>3.2-3.8 (m, 4H)<br>1.4-1.9 (m, 6H)<br>1.30 (t, 3H) |

- to be continued -

Table 1 (continued)

| Compound No. | IR | | NMR | |
|---|---|---|---|---|
| | $\nu_{max}$ ($cm^{-1}$) | Assignment | Solvent | δ (ppm) |
| 360 | 2210<br>1670 | Cyano<br>Carbonyl | $CDCl_3$ | 8.33 (s, 1H)<br>3.13 (q, 2H)<br>3.00 (q, 2H)<br>1.46 (t, 3H)<br>1.33 (t, 3H) |
| 362 | 2200<br>1650 | Cyano<br>Carbonyl | $CDCl_3$ | 7.93 (t, 1H)<br>4.40 (q, 2H)<br>3.00 (q, 2H)<br>1.50 (t, 3H)<br>1.30 (t, 3H) |
| 364 | 2200<br>1650 | Cyano<br>Carbonyl | $CDCl_3$ | 8.40 (s, 1H)<br>7.40 (s, 5H)<br>3.06 (q, 2H)<br>1.10 (t, 3H) |
| 330 | 2210<br>1710 | Cyano<br>Carbonyl | $CCl_4$ | 8.30 (s, 1H)<br>4.25 (q, 2H)<br>3.06 (q, 2H)<br>1.45 (t, 3H)<br>1.33 (t, 3H) |
| 332 | - | - | $CCl_4$ | 8.30 (s, 1H)<br>4.20 (q, 2H)<br>2.95 (t, 2H)<br>1.5-2.0 (m, 2H)<br>1.30 (t, 3H)<br>1.03 (t, 3H) |

- to be continued -

Table 1 (continued)

| Compound No. | IR | | NMR | |
|---|---|---|---|---|
| | $\nu_{max}(cm^{-1})$ | Assignment | Solvent | δ (ppm) |
| 334 | - | - | $CCl_4$ | 8.30 (s, 1H)<br>4.25 (q, 2H)<br>3.30 (t, 2H)<br>1.1-2.0 (m, 4H)<br>1.3 (t, 3H)<br>1.00 (t, 3H) |
| 336 | - | - | $CCl_4$ | 8.30 (s, 1H)<br>4.30 (q, 2H)<br>3.05 (t, 2H)<br>1.1-2.0 (m, 6H)<br>1.40 (t, 3H)<br>1.00 (t, 3H) |
| 338 | - | - | $CCl_4$ | 8.25 (s, 1H)<br>4.20 (q, 2H)<br>3.00 (t, 2H)<br>1.1-2.0 (m, 15H)<br>0.90 (t, 3H) |
| 365 | 2210<br>1670 | Cyano<br>Carbonyl | $CDCl_3$ | 7.95 (s, 1H)<br>7.36 (br, s, 5H)<br>4.30 (q, 2H)<br>1.40 (t, 3H) |
| 366 | - | - | $CDCl_3$ | 8.35 (s, 1H)<br>7.40 (s, 5H)<br>3.30 (t, 2H)<br>1.1-2.0 (m, 4H)<br>1.0 (t, 3H) |

- to be continued -

Table 1 (continued)

| Compound No. | IR | | NMR | |
|---|---|---|---|---|
| | $\nu_{max}(cm^{-1})$ | Assignment | Solvent | δ (ppm) |
| 368 | - | - | $CDCl_3$ | 7.95 (s, 1H)<br>7.20 (br, s, 5H)<br>4.32 (q, 2H)<br>4.15 (s, 2H)<br>1.40 (t, 3H) |
| 370 | - | - | $CDCl_3$ | 7.85 (s, 1H)<br>7.22 (br, s, 5H)<br>4.30 (t, 2H)<br>4.15 (s, 2H)<br>2.1-1.2 (m, 4H)<br>1.02 (t, 3H) |
| 372 | - | - | $CDCl_3$ | 8.40 (s, 1H)<br>7.20 (br, s, 5H)<br>4.15 (s, 2H)<br>3.04 (q, 2H)<br>1.10 (t, 3H) |
| 374 | - | - | $CDCl_3$ | 8.35 (s, 1H)<br>7.20 (br, s, 5H)<br>4.15 (s, 2H)<br>3.30 (t, 2H)<br>1.1-2.0 (m, 4H)<br>1.00 (t, 3H) |
| 380 | - | - | $CDCl_3$ | 7.42 (br, s, 5H)<br>3.95 (q, 2H)<br>2.61 (s, 3H)<br>1.28 (t, 3H) |

- to be continued -

Table 1 (continued)

| Compound No. | IR | | NMR | |
|---|---|---|---|---|
| | $\nu_{max}(cm^{-1})$ | Assignment | Solvent | δ (ppm) |
| 382 | - | - | $CDCl_3$ | 7.40 (br, s, 5H)<br>4.50 (q, 2H)<br>2.60 (q, 2H)<br>1.46 (t, 3H)<br>1.25 (t, 3H) |
| 390 | - | - | $CDCl_3$ | 7.80 (s, 1H)<br>5.5-6.0 (m, 1H)<br>4.20 (t, 2H)<br>3.5-4.0 (m, 1H)<br>0.8-2.1 (m, 15H) |
| 392 | - | - | $CCl_4$ | 8.30 (s, 1H)<br>5.5-6.0 (m, 1H)<br>3.5-4.2 (m, 1H)<br>3.10 (q, 2H)<br>0.8-1.8 (m, 11H) |
| 394 | - | - | $CDCl_3$ | 8.25 (s, 1H)<br>5.5-6.0 (m, 1H)<br>3.5-4.0 (m, 1H)<br>3.00 (t, 2H)<br>0.8-2.0 (m, 15H) |
| 400 | - | - | $CDCl_3$ | 7.70 (s, 1H)<br>4.20 (t, 2H)<br>3.35-3.75 (m, 4H)<br>0.8-1.9 (m, 13H) |
| 402 | - | - | $CDCl_3$ | 8.42 (s, 1H)<br>3.2-3.7 (m, 4H)<br>3.05 (q, 2H)<br>1.5-1.9 (m, 6H)<br>1.30 (t, 3H) |

- to be continued -

Table 1 (continued)

| Compound No. | IR | | NMR | |
|---|---|---|---|---|
| | $\nu_{max}$ (cm$^{-1}$) | Assignment | Solvent | δ (ppm) |
| 404 | - | - | $CDCl_3$ | 8.30 (s, 1H)<br>3.2-3.7 (m, 4H)<br>3.02 (t, 2H)<br>1.1-2.0 (m, 13H) |
| 408 | - | - | $CDCl_3$ | 7.70 (s, 1H)<br>6.5-7.2 (br, s, 2H)<br>4.25 (q, 2H)<br>1.30 (t, 3H) |
| 410 | 2210<br>1080 | Cyano<br>Carbonyl | $CDCl_3$ | 8.40 (s, 1H)<br>6.5-7.1 (br, s, 2H)<br>3.00 (q, 2H)<br>1.45 (t, 3H) |
| 440 | 1720<br>1560 | Carbonyl<br>Nitro | $CDCl_3$ | 7.8 (s, 1H)<br>4.40 (q, 2H)<br>4.26 (q, 2H)<br>1.52 (t, 3H)<br>1.28 (t, 3H) |
| 442 | 1720<br>1560 | Carbonyl<br>Nitro | $CDCl_3$ | 8.36 (s, 1H)<br>4.30 (q, 2H)<br>2.96 (q, 2H)<br>1.40 (t, 3H)<br>1.30 (t, 3H) |
| 476 | 1670 | Carbonyl | $CDCl_3$ | 9.15 (s, 1H)<br>8.40 (s, 1H)<br>7.1-7.5 (m, 5H) |

- to be continued -

Table 1 (continued)

| Compound No. | IR | | NMR | |
|---|---|---|---|---|
| | $\nu_{max}$ ($cm^{-1}$) | Assignment | Solvent | δ (ppm) |
| 486 | 1710 | Carbonyl | $CCl_4$ | 8.13 (s, 1H)<br>7.2-7.6 (m, 5H)<br>4.20 (q, 2H)<br>1.30 (t, 3H) |
| 504 | - | - | $CCl_4$ | 7.60 (s, 1H)<br>4.26 (q, 2H)<br>4.13 (q, 2H)<br>2.30 (s, 3H)<br>1.40 (t, 3H)<br>1.30 (t, 3H) |
| 506 | - | - | $CCl_4$ | 7.80 (s, 1H)<br>4.30 (t, 2H)<br>4.20 (q, 2H)<br>2.31 (s, 3H)<br>0.8-0.9 (m, 10H) |
| 512 | 1700-1710 | Carbonyl | $CCl^4$ | 7.30 (s, 1H)<br>4.20 (q, 2H)<br>3.90 (q, 2H)<br>3.92 (q, 2H)<br>1.0-1.5 (m, 9H) |
| 515 | 1700 | Carbonyl | $CDCl_3$ | 8.1 (s, 1H)<br>4.0-4.5 (m, 4H)<br>2.92 (q, 2H)<br>1.05-1.50 (m, 9H) |
| 517 | 1660-1700 | Carbonyl | $CDCl_3$ | 8.15 (s, 1H)<br>3.9-4.5 (m, 4H)<br>3.2-5.0 (m, 2H)<br>1.1-1.5 (m, 6H) |

- to be continued -

Table 1 (continued)

| Compound No. | IR | | NMR | |
|---|---|---|---|---|
| | $\nu_{max}(cm^{-1})$ | Assignment | Solvent | δ (ppm) |
| 518 | 1650-1700 | Carbonyl | $CDCl_3$ | 7.3-9.6 (br, s, 3H)<br>3.95-4.5 (m, 4H)<br>1.1-1.5 (m, 6H) |

Formulation Example 1

One part of active compound No. 330 of this invention was added to 225 parts of a mixture of acetone and water (volume ratio 1:1), and 0.5 part of a nonionic surface-active agent (Sorpol-2680, a tradename) to prepare a solution.

Formulation Example 2

One part of active compound No. 318 of this invention was added to 250 parts of water, and 0.5 part of a nonionic surface-active agent was added (Alsoap-30, a tradename) to prepare a solution.

Formulation Example 3

A mixture of 0.3 part of active compound No. 320 of this invention, 0.63 part of a xylene-isophorone mixture and 0.07 part of a nonionic surface-active agent (Sorpol 800-A, a tradename) was diluted with water to a predetermined concentration.

Formulation Example 4

0.1 Part of active compound No. 324 of this invention, 0.87 part of bentonite and 0.03 part of a nonionic surface-active agent (Sorpol 800-A, a tradename) were fully mixed to form a wettable powder which was diluted with water to a predetermined concentration.

Example 1

The active compounds of this invention indicated in Table 2 were formulated in accordance with the above Formulation Examples.

Seeds of the weeds indicated in Table 2 were

sown, and after emergence, grown for 2 to 3 weeks.

Each of the formulations containing the active compounds of this invention was applied to these weeds three times at an interval of about 30 minutes in an amount of about 0.5 to 1 $g/m^2$ for each application as the amount of the active ingredient. Thereafter, without applying the formulation, the weeds were grown for one week. The results are shown in Table 2.

Annual fleabane (Erigeron annus) is an annual broad-leaved plant; fingergrass (Digitaria sanguinalis) is an annual narrow-leaved plant; broadleaf plantain (Plantago asicatica) is a perennial broad-leaved plant; and nut grass (Cyperus rotundus) is a perennial narrow-leaved plant.

The results given in Table 2 demonstrate that the active compounds of the invention shown in Table 2 have excellent herbicidal activity against any of these plants.

Table 2

| No. of active compound | Herbicidal activity | | | |
|---|---|---|---|---|
| | Annual fleabane | Finger-grass | Broadleaf plantain | Nut grass |
| 322 | 5 | 5 | 5 | 5 |
| 324* | 5 | 5 | 5 | 5 |
| 326 | 5 | 5 | 5 | 5 |
| 330* | 5 | 5 | 5 | 5 |
| 318* | 5 | 5 | 4 | 5 |
| 138 | 5 | 5 | 5 | 3 |
| 349* | 5 | 4 | 5 | 3 |
| 350* | 5 | 3 | 4 | 3 |
| 442 | 3 | 4 | 3 | 4 |
| 360* | 4 | 4 | 3 | 3 |
| 320* | 5 | 2 | 3 | 2 |
| 320** | 4 | 1 | 3 | 1 |
| 362 | 3 | 2 | 2 | 2 |

In Table 2, the single asterisk shows that the herbicidal activity was examined 5 days after the application of the active compound, and the double asterisk shows that the herbicidal activity was examined 2 days after the application. For the non-asterisked compounds, the herbicidal activities were examined one week after the application. The same designations apply to Tables 3 to 6 below.

Example 2

In the same way as in Example 1, the active compounds shown in Table 3 were tested, and the results are shown in the same table. These active compounds qere found to exhibit activity against annual plants and perennial broad-leaved plants.

Table 3

| No. of active compound | Herbicidal activity | | | |
|---|---|---|---|---|
| | Annual fleabane | Finger-grass | Broadleaf plantain | Nut grass |
| 144* | 5 | 5 | 5 | 1 |
| 353 | 5 | 4 | 5 | 1 |
| 324* | 5 | 4 | 3 | 1 |
| 332* | 4 | 5 | 3 | 1 |
| 334* | 3 | 5 | 3 | 1 |
| 352* | 5 | 3 | 4 | 1 |
| 380 | 5 | 3 | 3 | 1 |
| 394 | 3 | 4 | 2 | 1 |
| 298 | 2 | 2 | 3 | 1 |

Example 3

The active compounds indicated in Table 4 were tested in the same way as in Example 1, and the results are shown in the same table. These active compounds were found to exhibit activity against annual plants.

Table 4

| No. of active compound | Herbicidal activity | | | |
|---|---|---|---|---|
| | Annual fleabane | Finger-grass | Broadleaf plantain | Nut grass |
| 184* | 5 | 3 | 2 | 1 |
| 358 | 5 | 3 | 1 | 1 |
| 396 | 4 | 4 | 2 | 1 |
| 382 | 4 | 3 | 2 | 2 |
| 524 | 3 | 4 | 1 | 1 |
| 506 | 3 | 3 | 1 | 1 |
| 366 | 3 | 3 | 1 | 1 |
| 404 | 3 | 3 | 1 | 1 |

Example 4

The active compounds indicated in Table 5 were tested in the same way as in Example 1, and the results are shown in the same table. These active compounds were found to exhibit activity against the annual narrow-leaved plant.

Table 5

| No. of active compound | Herbicidal activity | | | |
|---|---|---|---|---|
| | Annual fleabane | Finger-grass | Broadleaf plantain | Nut grass |
| 336* | 1 | 5 | 2 | 1 |
| 338* | 1 | 5 | 1 | 1 |
| 515* | 2 | 4 | 2 | 1 |
| 347 | 1 | 3 | 1 | 1 |
| 486 | 1 | 3 | 1 | 1 |

Example 5

The active compounds indicated in Table 6 were tested in the same way as in Example 1, and the results are shown in the same table. These active compounds were found to exhibit activity against the annual broad-leaved plant.

Table 6

| No. of active compound | Herbicidal activity | | | |
|---|---|---|---|---|
| | Annual fleabane | Finger-grass | Broadleaf plantain | Nut grass |
| 106* | 5 | 2 | 2 | 1 |
| 112* | 5 | 2 | 2 | 1 |
| 348* | 5 | 1 | 1 | 1 |
| 357* | 5 | 1 | 1 | 1 |
| 406* | 5 | 1 | 2 | 2 |
| 182* | 4 | 2 | 2 | 1 |
| 294* | 4 | 1 | 2 | 2 |
| 365* | 4 | 1 | 1 | 1 |
| 370 | 4 | 2 | 2 | 1 |
| 504* | 4 | 1 | 2 | 2 |
| 316 | 3 | 1 | 1 | 1 |
| 344 | 3 | 1 | 1 | 1 |
| 368 | 3 | 2 | 1 | 1 |
| 412 | 3 | 1 | 1 | 1 |

Example 6

Upland farm soil was put in pots (10 cm x 10 cm), and seeds of annual fleabane were sown. When they grew to a height of 8 to 10 cm, 2 ml of a chemical prepared in accordance with the following formulation was sprayed onto the plants. Ten days after the spraying, the growth of the plants was measured by their height.

Formulation of the chemical

| | |
|---|---|
| Compound No. 330 of the invention | 0.1 mg |
| Surface-active agent (Sorpol 800-A) | 23 mg |
| Isophorone | 100 mg |
| Xylene | 133 mg |
| Water | 5.5 ml |

Likewise, annual fleabane grown to a height of 8 to 10 cm were grown without treating it with the herbicidal chemical, and its height was measured 10 days

later.

The results are shown below.

| | Height 10 days after the spraying |
|---|---|
| Treated plant | 12-13 cm |
| Non-treated plant | 20-25 cm |

Example 7

The active compounds of this invention show herbicidal activity and plant growth modifying activity against weeds as shown in Examples 1 to 6. These compounds are useful for controlling weeds which grow in areas where useful plants are cultivated. Table 7 below gives a list of the active compounds of this invention which do not substantially affect the growth of rice, radish, maize, cucumber and soybean which are useful crops.

Table 8

| Rice | Radish | Maize | Cucumber | Soybean |
|---|---|---|---|---|
| 106 | 106 | 106 | 294 | 112 |
| 182 | 112 | 112 | 316 | 184 |
| 184 | 294 | 182 | 344 | 334 |
| 294 | 316 | 184 | 348 | 338 |
| 316 | 322 | 294 | 357 | 344 |
| 320 | 334 | 298 | 358 | 348 |
| 322 | 336 | 320 | 365 | 352 |
| 324 | 338 | 322 | 366 | 357 |
| 326 | 344 | 338 | 368 | 358 |
| 332 | 347 | 344 | 370 | 362 |
| 334 | 348 | 347 | 374 | 365 |
| 336 | 357 | 348 | 380 | 368 |
| 338 | 358 | 353 | 394 | 402 |
| 344 | 362 | 357 | 402 | 406 |
| 347 | 365 | 358 | 406 | 412 |
| 348 | 366 | 362 | 412 | 486 |
| 352 | 368 | 365 | 504 | 504 |
| 353 | 370 | 368 | | 524 |
| 357 | 374 | 370 | | |
| 358 | 394 | 380 | | |
| 365 | 402 | 394 | | |
| 366 | 404 | 396 | | |
| 368 | 406 | 404 | | |
| 370 | 412 | 406 | | |
| 380 | 442 | 412 | | |
| 382 | 486 | 442 | | |
| 394 | 504 | 504 | | |
| 396 | 515 | 506 | | |
| 402 | 524 | 524 | | |
| 404 | | | | |
| 406 | | | | |
| 412 | | | | |

- to be continued -

Table 8 (continued)

| Rice | Radish | Maize | Cucumber | Soybean |
|---|---|---|---|---|
| 442 | | | | |
| 504 | | | | |
| 506 | | | | |
| 515 | | | | |
| 524 | | | | |

What is claimed is:

1. A herbicide comprising as an active ingredient an α,β-unsaturated compound represented by the following formula (I)

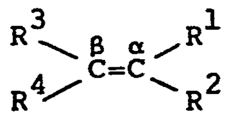

..... (I)

wherein

$R^1$ and $R^2$ are identical or different and each represents

CN,

$NO_2$,

a halogen atom,

a $C_{1-25}$ alkoxy or alkylthio group,

a group of the formula $-\underset{\overset{\|}{O}}{C}Z^1$ in which $Z^1$ represents a $C_{1-25}$ alkyl group or a phenyl group, which phenyl group may be substituted by 1 to 5 halogen atoms or 1 to 5 $C_{1-5}$ alkyl groups,

a group of the formula $-\underset{\overset{\|}{X}}{C}XZ^2$ in which X's, independently from each other, represent an oxygen or sulfur atom and $Z^2$ represents a hydrogen atom, one equivalent of a cation, a $C_{1-25}$ alkyl group, a phenyl group or a phenyl-($C_{1-25}$ alkyl) group, the phenyl moiety of which may be substituted by 1 to 5 halogen atoms or 1 to 5 $C_{1-5}$ alkyl groups, or

a group of the formula $-\underset{\overset{\|}{X}}{C}N\begin{matrix}Z^3\\Z^4\end{matrix}$ in which X is as defined above, and $Z^3$ and $Z^4$, independently from each other, represent a hydrogen atom, a $C_{1-25}$ alkyl group, a phenyl($C_{1-25}$ alkyl) group or a triazolyl group, which triazolyl group may be substituted by a $C_{1-5}$ alkyl group, a phenyl group or a $C_{2-5}$ carboacyl group, or

$Z^3$ and $Z^4$ may be bonded to each other to form a 5- or 6-membered ring together with the nitrogen atom to which they are bonded;

provided that $R^1$ and $R^2$ do not simultaneously represent the same member of the class consisting of $NO_2$, halogen atoms, $C_{1-25}$ alkoxy or alkylthio groups and groups $-\underset{\overset{\|}{X}}{C}Z^2$ in which $Z^2$ is a hydrogen atom;

$R^3$ represents

a group of the formula $Z^5X-$ in which $Z^5$ represents a hydrogen atom or a $C_{1-25}$ alkyl group and X is as defined above,

a group of the formula $Z^6O\underset{\overset{\|}{O}}{C}-$ in which $Z^6$ represents a hydrogen atom, one equivalent of a cation or a $C_{1-25}$ alkyl group,

a group of the formula $Z^7O\underset{\overset{\|}{O}}{C}-$ in which $Z^7$ represents a hydrogen atom or a $C_{1-25}$ alkyl group, or

a phenylthio, phenyl($C_{1-25}$ alkylthio) or phenyl($C_{1-25}$ alkoxy) group, the phenyl moiety of which may be substituted by 1 to 5 halogen atoms or 1 to 5 $C_{1-5}$ alkyl groups;

$R^4$ represents

a hydrogen atom,

a $C_{1-25}$ alkyl group,

a 3- to 6-membered cycloalkyl group,

a phenyl($C_{1-25}$ alkyl) group the phenyl moiety of which may be substituted by 1 to 5 halogen atoms or 1 to 5 $C_{1-5}$ alkyl groups, or

the same groups as defined for $R^3$ above; and

$R^3$ and $R^4$ may be bonded to each other to form a 5- or 6-membered carbocyclic ring together with the carbon atom to which they are bonded;

provided that $R^3$ and $R^4$ do not simultaneously represent the same member of the class consisting

of HO-, HS-, $C_{1-25}$ alkylthio groups, $HO\overset{O}{\overset{\|}{C}}-$, $(C_{1-25}$ alkyl$)\overset{O}{\overset{\|}{C}}O-$ and phenyl($C_{1-25}$ alkylthio) groups, and when $R^3$ is HO-, $R^4$ does not represent a hydrogen atom; and

further provided that $R^1$ or $R^2$ and $R^3$ or $R^4$ may be bonded to each other to form an acid anhydride group and that the $C_{1-5}$ alkyl and $C_{1-25}$ alkyl groups in the foregoing definitions may be interrupted by -O-, -S- or $-\overset{|}{N}Z^8$ in which $Z^8$ represents a hydrogen atom or a $C_{1-10}$ alkyl group.

2. The herbicide of claim 1 wherein $R^1$ and $R^2$ are identical or different and each represents CN, $NO_2$, a halogen atom, a group of the formula $-\underset{X}{\underset{\|}{C}}Z^2$ in which $Z^2$ and X are as defined above provided tnat at least one of the two X's is an oxygen atom, or a group of the formula $-\underset{O}{\underset{\|}{C}}N\begin{matrix}Z^3\\Z^4\end{matrix}$ in which $Z^3$ and $Z^4$ are as defined above, or $R^1$ or $R^2$ is bonded to $R^4$ to form an acid anhydride group, provided that $R^1$ and $R^2$ do not simultaneously represent the same member of the class consisting of $NO_2$, halogen atoms and groups of the formula $-\underset{X}{\underset{\|}{C}}Z^2$ in which $Z^2$ is a hydrogen atom.

3. The herbicide of claim 1 or 2 wherein $R^3$ is as defined in claim 1, $R^4$ represents a hydrogen atom, a $C_{1-25}$ alkyl group, a group of the formula $Z^{51}X-$ in which $Z^{51}$ represents a $C_{1-25}$ alkyl group and X is as defined, or a phenyl($C_{1-10}$ alkylthio) group, provided that the $C_{1-25}$ alkyl group may be interrupted by -O- or -S-, and $R^3$ and $R^4$ do not simultaneously represent the member of the class consisting of $C_{1-25}$ alkylthio groups and phenyl($C_{1-10}$ alkylthio) groups.

4. The herbicide of claim 1 comprising as an active ingredient an α,β-unsaturated compound represented

by the formula (I)-1

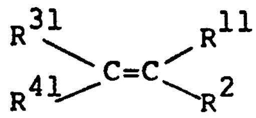

..... (I)-1

wherein

$R^{11}$ represents CN, a halogen atom, a group of the formula $-\underset{\underset{O}{\|}}{C}Z^1$ in which $Z^1$ is as defined, or a group of the formula $-\underset{\underset{O}{\|}}{C}OZ^2$ in which $Z^2$ is as defined above;

$R^2$ is as defined above,

provided that $R^{11}$ and $R^2$ do not simultaneously represent the same member of the class consisting of halogen atoms and -COOH;

$R^{31}$ represents a group of the formula $Z^{51}X$- in which $Z^{51}$ and X are as defined above or a phenyl-($C_{1-10}$ alkylthio) group;

$R^{41}$ represents a hydrogen atom, or a $C_{1-25}$ alkyl group which may be interrupted by -O- or -S-; and

$R^{31}$ and $R^{41}$ may be bonded to each other to form a 6-membered carbocyclic ring together with the carbon atom to which they are bonded.

5. The herbicide of claim 4 wherein $R^2$ represents CN, $NO_2$, a halogen atom, a group of the formula $-\underset{\underset{X}{\|}}{C}XZ^2$ in which $Z^2$ and X are as defined above provided that at least one of the two X's is an oxygen atom, or a group of the formula 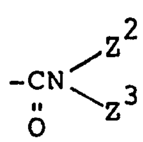 in which $Z^2$ and $Z^3$ are as defined above, and $R^2$ may be bonded to $R^{31}$ or $R^{41}$ to form an acid anhydride group, provided that $R^{11}$ and $R^2$ do not simultaneously represent the same member of the class consisting of halogen atoms and -COOH.

6. The herbicide of claim 1 which further comprises a carrier.

7. The herbicide of claim 1 which further comprises a carrier and a surface-active agent.

8. The herbicide of claim 1, 6 or 7 wherein the amount of the α,β-unsaturated compound of formula (I) is 0.01 to 99% by weight.

9. A method for combating weeds, which comprises applying the herbicide of claim 1 to weeds or their habitat.

10. The method of claim 9 wherein the rate of application of the α,β-unsaturated compound of formula (I) is 10 g to 200 kg/hectare.

11. A plant growth regulator comprising an α,β-unsaturated compound of formula (I) given in claim 1 as an active ingredient.